# EUROPEAN PATENT SPECIFICATION

(11) **EP 0 756 872 B1**
(45) Date of publication and mention of the grant of the patent: **15.05.2002**
(21) Application number: 96202193.7
(22) Date of filing: 05.08.1996
(51) Int. Cl.: A61K 38/48

(54) **Collagenase for inducing cervical ripening**
Collagenase zur Zervikalreifung
Collagénase pour le mûrissement cervical

(30) Priority: 04.08.1995 ZA 9506513
(43) Date of publication of application: 05.02.1997
(73) Proprietor: Lebovitz, Shamir, 67456 Tel Aviv (IL)
(72) Inventor: Lebovitz, Shamir, 67456 Tel Aviv (IL)
(74) Representative: Smulders, Theodorus A.H.J., Ir.

(56) References cited:
- EP-A- 0 543 476
- US-A- 3 678 158
- US-A- 4 174 389
- US-A- 4 338 300
- AMERICAN JOURNAL OF OBSTETRICS AND GYNECOLOGY, vol. 159, no. 4, 1988, pages 971-976, XP000608485 RAJABI, M.R. ET AL: "Elevated tissue levels of collagenase during dilation of uterine cervix in human parturition"
- ARCHIVES OF GYNECOLOGY AND OBSTETRICS, vol. 248, no. 2, 1990, pages 75-80, XP000608743 OSMERS, R. ET AL: "Collagenase activity in the cervix of non-pregnant and pregnant woman"

## Description

This invention relates to the use of collagenase in the preparation of a substance or composition for softening or ripening the uterine cervix of female mammals, including humans.

The invention relates to the softening or ripening the uterine cervix of female mammals, including humans, by administering an effective amount of collagenase.

The invention can beneficially be used, for example, in induction of labor and in termination of pregnancy.

Collagen is a naturally occurring fibrous protein found in humans and animals. Collagen is one of the most abundant proteins in mammals and comprised various naturally occurring amino acids, e.g. glycine, alanine, proline and hydroxyproline. The uterine cervix contains collagen which is'degraded by the collagenase enzyme, particularly collagen type I and collagen type III.

EP-A-543 476 discloses the use of interleukin-8 for inducing cervical ripening. This document also mentions the current preferred method of cervical ripening with prostaglandin E₂.

The present invention is based on the finding that during labor and until the end of the delivery, the amount of collagen in the cervix is usually significantly reduced. The collagen in the cervix and lower segment of the cervix is degraded as a result of an increase in the amount of collagenase (molecular weight of approximately 70,000 dalton). The ratio of undissolved hydroxyproline in collagen to the total amount of protein in collagen generally is about 0,75 in women not in pregnancy. Such ratio is generally about 0,3 in women in active labor.
This is an indication of the degree of degradation of collagen caused by collagenase in the lower segment of the cervix and the uterus in women in or nearing labor. Reduction in the amount of collagen results in a softening or ripening of the cervix, which allows dilation thereof and thereby facilitates birth.

Various methods can be employed to determine whether or not the cervix is in a "favourable condition". One such method is by means of measuring the Bishop score of the cervix of a woman in labor, e.g. to determine whether or not the cervix of a woman has reached a favourable condition of softness of ripeness prior to delivery. A bishop score on a scale of 0-10 is carried out on the cervix. The parameters of the cervix measured to determine the Bishop score are set out hereunder:
Length of cervix - 0-3 points
Dilation of cervix - 0-3 points
Consistency of cervix - 0-2 points
Position of cervix (anterior, mid and posterior) - 0-2 points (i.e. posterior = 0, anterior = 2)
Station of leading part (head breach) - 0-3 points

A Bishop score >6 indicates that the cervix is in a "favourable condition" for dilation and a Bishop score <4 indicates that the cervix is in an "unfavourable condition".

However, on occasion induction of labor is required when the cervix is in an "unfavourable condition", and maintains the cervix in a firmer unripened condition which does not readily allow dilation thereof.

Furthermore, access into and from the uterus is not necessarily limited to natural childbirth. For example, in various clinical situations, access into the uterus is required for purposes of curettage, while termination of pregnancy requires passage through the cervix of an embryo of foetus. It will be appreciated that in such cases, the cervix is unlikely to be in a favourable condition.

It will be appreciated that the extent to which dilation of the cervix is required for childbirth is greater than what generally is required for early termination of pregnancy or curettage.

The present invention solves this problem by reducing the collagen content, since it has been found that in case of the "unfavourable condition", the collagen content has not been reduced by natural biological processes of the pregnant female mammal body. More in particular, the present invention comprises administering an effective amount of a composition comprising collagenase to a female mammal.

More in detail, the present invention relates to the use of collagenase in the manufacture of a medicament for softening and ripening the cervix of the uterus of a female mammal; the use of collagenase in the manufacture of a medicament to induce labour in a female mammal and the use of collagenase in the manufacture of a medicament to terminate pregnancy in a female mammal.

Moreover, the present invention relates to the treatment of female mammals for softening or ripening the cervix of the uterus, by administering an effective amount of a composition comprising collagenase to said female mammal. In other words, collagenase is used in a method comprising including an effective amount of collagenase in the preparation of a composition suitable for softening or ripening the uterine cervix of female mammals, for inducing labour in female mammals and also for terminating pregnancy in female mammals.

The term "effective amount" of collagenase or of the substance or composition should be understood as being sufficient to give the desired result while being non-toxic to the female mammal. Furthermore, "softening or ripening" of the cervix should be understood as meaning such change in the collagen content of the cervix as will allow desired dilation thereof.

By stimulating, enhancing of increasing the collagenase activity or amount of collagenase in the cervix of pregnant females, according to the method of the present invention, the cervix can be softened or ripened when desired, e.g. prior to termination of pregnancy or induction of labour. In other words, the substance or composition can be administered to female mammals to provide at least some of the collagenase necessary to degrade collagen in the cervix and thereby ripen or soften said cervix. The collagenase administered may augment any naturally produced collagenase present in the cervix and/or stimulate the production of naturally occurring collagenase in the cervix.

Naturally, routine experimentation can be used to optimise the effective amount of collagenase required to be used in a substance or composition in accordance with the invention.

The substance or composition comprising the collagenase may be administered in various forms and by various routes, e.g. subcutaneously, intramuscularly, intravenously, intracervically, etc., and therefore the substance or composition may be provided in the form of a liquid or a solid formulation e.g. pills, gels, capsules. The preferred method of administration is by injection into a lip of the cervix as a liquid, and preferably by injection into both lips.

the particular pharmaceutical carrier used will vary, depending on the form of the substance or composition and the intended method of administration.

The substance of composition may be administered by injection into the cervix in such amount as will provide collagenase to the cervix at a concentration in the range of 10-15 I.U. per gram of cervical tissue, e.g. 12 I.U. per gram cervical tissue and preferably provide a serum level of collagenase of from about 60 to about 80 nanograms per millilitre.

The substance or composition and method according to the present invention may be administered in conjunction or in combination with other agents or methods which heretofore have been used in an endeavour to soften or ripen the cervix. These include the use of hydrophilic organic material obtained from seaweed (Laminaria japonicum); synthetic hydrophilic material, e.g. such as that sold under the trade mark Lamicel; prostaglandins in gel or tablet form; and intravenously administered oxytocin. Furthermore, collagenase may be used in combination with at least one other enzyme such as yaloronidase, elastase heparin sulphatase, dermatin sulphatase or the like, the other enzyme(s) facilitating the degradation of mucopolysaccharides in the cervix and, the collagenase degrading the collagen.

Collagenase enzyme for use in the present invention is readily obtainable. It is synthesised commercially using the bacterium Clostridium hystoliticum.

The invention is now described with reference to the following proposed non-limiting examples of a method of treatment of human female subjects using a substance or composition in accordance with the invention.

### EXAMPLE 1

### Termination of pregnancy in the first trimester

Thirty women undergoing curettage in the first trimester of pregnancy were recruited for a study. The study was a double blind randomised study with collagenase enzyme at a concentration of 10-15 I.U. per gram cervical tissue being administered to fifteen of the women, and saline at a concentration of 0,9% by mass being administered to the remaining fifteen women.

2ml injection ampoules were prepared so that neither the medical nor nursing staff nor the patient were able to identify whether or not the ampoules contained saline of collagenase. The ampoules were not marked with a code so that the person running or controlling the test was unable to identify which patients have been given saline and which have been given collagenase treatment (a double blind study).

Twelve hours before dilatation and curettage, 2ml of he collagenase or saline solution was injected at the anterior and posterior lip of the cervix, 1ml being injected to each lip at the sic and the twelve o'clock positions. Serum, prostaglandin F₂α (i.e. pgF₂α) and prostaglandin Eα (i.e. pgEα) levels were measured before injecting the cervix and again before termination of pregnancy by using Amerlex-magnetic reparation obtainable from the Weil organisation of South Africa.

At the time of the curettage, the pressure required to insert Hegar dilation instruments sizes 6-12 was measured by the pressure measuring instrument used by N.D. Goldstuck according to Goldstuck N.D., Holloway - Effects of recent childbirth and lactation. 159-164, 1980. The duration of the induction or the delivery interval of labour as well as the estimated blood loss were recorded in each case. The method of the present invention showed significant better results.

### EXAMPLE 2

### Termination of pregnancy in the second trimester

Thirty women undergoing termination of pregnancy in the second trimester were recruited for this study. The identical dosages of collagenase or saline were injected into the cervix as described in Example 1 above. The Bishop score was measured prior to injection and again after twelve hours and recorded. Routine termination of pregnancy was performed by injecting pgEα intracervically or pgF₂α extra-amniotically. The interval between induction and delivery was recorded. The termination of pregnancy progressed significantly more smoothly when using the preparation according to the invention.

### EXAMPLE 3

### Women before undergoing induction of labour in the third trimester

This may be necessary because of the mother's condition (e.g. hypertension, diabetes, etc.) or to induce labour post-term (42 completed weeks) or in term due to foetal causes (intra-uterine growth retardation (I.U.G.R) or non-reactive non-stress test (N.S.T.), etc.).

Thirty women undergoing induction of labour in the third trimester were used for this study. The same method as carried out in Example 1 and 2 above was applied, resulting in a more nature-like labour behaviour when using collagenase.

### EXAMPLE 4

### Women before undergoing hysterectomy

Thirty women undergoing hysterectomy were used for this study. Collagenase and saline were injected 21 hours prior to hysterectomy in accordance with the method as described in Example 1 above. Cervical resistance was then measured as described by Goldstuck. A hysterectomy specimen from each woman was semi-quantitatively histologically examined for collagen content and compared with the saline control group to ensure that there are no adverse side-effects and to measure the collagenase concentration in the blood. The collagenase concentration was higher in collagenase-treated women, while the cervical resistance was found lower.

The present invention thus provides for the use of collagenase in obstetrics and gynaecology to soften and ripen the cervix prior to termination of pregnancy or induction of labour in situations where the cervix is not in a favourable condition.

Conventional procedures in which mechanical dilation of the cervix is effected by dilators with increasing diameters, can cause tearing or damage to the cervix.

Induction of labour with prostaglandins locally and oxytocin intravenously can fail if the cervix is not in a favourable condition and may also be hazardous or toxic to the female if large doses are required. An advantage of using collagenase is that it comprises a naturally occurring enzyme that is physiologically compatible with the female's biochemistry and is generally non-toxic if used in prescribed dosages.

The use of collagenase will facilitate induction of labour and termination of pregnancy and other procedures such as curettage, and is expected to minimise the incidence of caesarian sections which heretofore have needed to be performed.

The use of collagenase is also expected to minimise or reduce damage to the uterine cervix caused during abortions using Hegar dilators, and to reduce cervix incompetence caused by any damage to the cervix during such operations.

## Claims

1. Use of collagenase in the manufacture of a medicament for softening and ripening the cervix of the uterus of a female mammal.

2. Use of collagenase in the manufacture of a medicament to induce labor in a female mammal.

3. Use of collagenase in the manufacture of a medicament to terminate pregnancy in a female mammal.

4. Use of any one of the preceding claims, wherein the female mammal is human.

## Patentansprüche

1. Verwendung von Collagenase zur Herstellung eines Medikaments zum Weichmachen und Reifen des Gebärmutterhalses eines weiblichen Säugers.

2. Verwendung von Collagenase zur Herstellung eines Medikaments zur Geburtseinleitung bei einem weiblichen Säuger.

3. Verwendung von Collagenase zur Herstellung eines Medikaments zur Schwangerschaftsunterbrechung bei einem weiblichen Säuger.

4. Verwendung nach irgendeinem der vorhergehenden Ansprüche, worin der weibliche Säuger ein Mensch ist.

## Revendications

1. Utilisation de collagène dans la fabrication d'un médicament pour le ramollissement et l'ouverture du col de l'utérus d'un mammifère femelle.

2. Utilisation de collagène dans la fabrication d'un médicament pour provoquer le travail chez un mammifère femelle.

3. Utilisation de collagène dans la fabrication d'un médicament pour finir la grossesse chez un mammifère femelle.

4. Utilisation selon l'une quelconque des revendications précédentes, dans laquelle le mammifère femelle est un être humain.
